# EUROPEAN PATENT SPECIFICATION

(11) **EP 0 513 127 B1**
(45) Date of publication and mention of the grant of the patent: **19.07.1995**
(21) Application number: 91903548.5
(22) Date of filing: 30.01.1991
(51) Int. Cl.: A61K 9/12, A61K 9/72

(54) **PROPELLANT COMPOSITIONS**
TREIBMITTELZUBEREITUNGEN
COMPOSITIONS D'AGENTS PROPULSEURS

(30) Priority: 02.02.1990 GB 9002351; 31.10.1990 GB 9023655; 05.12.1990 GB 9026476
(43) Date of publication of application: 19.11.1992
(73) Proprietor: FISONS plc, Ipswich Suffolk IP1 1QH (GB)
(72) Inventor: STEELE, Gerald, Leicestershire LE11 3HN (GB); SOMANI, Asit, Loughborough, Leicestershire LE11 1JR (GB); LIM, Joseph, Geok, Paan, Leicestershire LE12 9QR (GB)
(74) Representative: Wright, Robert Gordon McRae
(86) International application number: PCT/GB91/00133
(87) International publication number: WO 91/11173

(56) References cited:
- WO-A-90/07333
- US-A- 3 490 923
- US-A- 4 174 295
- US-A- 4 352 789

## Description

This invention relates to pressurised aerosol compositions, in particular compositions of inhalation medicaments.

Pressurised aerosols for the administration of medicaments, and indeed for other applications, conventionally contain one or more liquified chlorofluorocarbons (CFC's) as propellant. Such materials are suitable for use in such applications since they have the right vapour pressures (or can be mixed in the right proportions to achieve a vapour pressure in the right range) and are essentially taste- and odour-free.

In recent years there has been increasing concern about the depletion of the ozone layer in the upper atmosphere. This is believed to be due to the release into the atmosphere of CFC's and has led to a search for alternative agents for use in all applications of CFC's. To this end, aerosols for many applications are now pressurised using pressurised gases such as nitrogen or hydrocarbons. However, such propellants are generally not suitable for use in the administration of inhalation medicaments since they are toxic and/or the pressure within the canister falls each time the device is used which leads to unreproducible dosing.

The use of hydrofluorocarbons as aerosol propellants has also been suggested. European Patent Application 0 372 777, published after the earliest priority date of this application, states that the use of the hydrofluorocarbon propellant 134a and drug as a binary mixture or in combination with a conventional surfactant such as sorbitan trioleate does not provide formulations having suitable properties for use with pressurised inhalers and suggests that satisfactory formulations may be made by adding a compound having a higher polarity than propellant 134a, such as pentane or ethanol. It is stated that the addition of a compound of higher polarity than propellant 134a to propellant 134a provides a mixture in which increased amounts of surfactant may be dissolved compared to their solubility in propellant 134a alone. It is further stated that the presence of increased amounts of solubilised surfactant allows the preparation of stable, homogenous suspensions of drug particles. The use of such co-solvents is undesirable since they may have unsuitable properties, for example, they may be flammable and/or toxic.

US Patent No 4352789 suggests the use of perfluorinated surfactants which are insoluble in CFC or perfluorinated propellants as a coating for finely divided medicament to be formulated in CFC or perfluorinated propellants.

Surprisingly, we have now found that mixtures of hydrofluorocarbons and fluorinated surfactants have properties which render them suitable for use as propellant systems for aerosol compositions.

Thus, according to the invention there is provided a pressurised aerosol composition comprising a liquified hydrofluorocarbon propellant having dispersed therein a medicament; and a fluorinated surfactant dissolved in the propellant; characterised in that the solvent for the fluorinated surfactant consists essentially of the hydrofluorocarbon propellant.

The compositions according to the invention are advantageous in that the solubility of the surfactant is such as to ensure good dispersion of the medicament and smooth operation of the aerosol valve. In particular, and in contrast to EP-A-0 372 777, the surfactants which characterise the present invention are sufficiently soluble in hydrofluorocarbons to enable them to be used without the presence of an additional substance as co-solvent.

The propellant mixtures of the present invention may also be advantageous in that they are substantially taste- and odour-free and have suitable vapour pressures for the administration of medicaments by inhalation, yet are environmentally safe and acceptable, especially when compared with compositions including chlorofluorocarbons. In addition, they may be less irritant than corresponding compositions including conventional surfactants such as oleic acid and sorbitan trioleate.

A wide range of fluorinated surfactants may be used in the compositions of the present invention. The surfactant may be perfluorinated or otherwise.

Perfluorinated surfactants which may be used include ionic surfactants, both anionic and cationic, eg perfluorinated alcohol phosphate esters and their salts, perfluorinated sulphonamide alcohol phosphate esters and their salts, and perfluorinated alkyl sulphonamide alkylene quaternary ammonium salts. However, we prefer surfactants which are non-ionic.

Other surfactants may be used which, while not perfluorinated as such, contain at least one perfluorinated alkyl group.

We prefer surfactants which contain at least one (CF₂) group, more preferably from 2 to 60, eg 5 to 20 such groups.

We prefer surfactants which contain one or more ether or carboxylic ester linkages, more preferably from 2 to 60, eg 4 to 10 such linkages. We particularly prefer compounds which contain both ether and ester linkages.

We prefer surfactants which contain at least one (CH₂) group, more preferably from 2 to 60, eg 5 to 20 such groups. We further prefer surfactants which contain at least one (OCH₂CH₂) group, more preferably from 2 to 30, eg 3 to 10 such groups.

Preferred non-ionic surfactants include, for example fluorinated alcohols, esters, amides, N-oxides or sulphonamides. We particularly prefer polyfluoroalkyloxyethylenes of the general formula CₘF₂ₘ₊₁CH₂(OC₂H₄)ₙOH in which m is an integer from 7 to 18 and n is an integer from 2 to 6. Other preferred surfactants include:

(CF₃)₂CFO(CF₂)_{z}CONH(CH₂)₃N(O)(CH₃)₂,

(CF₃)₂CFOCF₂CF₂CH₂CH₂(0CH₂CH₂)_{z}OH,

in which z is an integer from about 2 - 20,

CF₃CF₂CF₂O(CF(CF₃)CF₂CF₂O)ₙCF₂CF₂CF₃,

in which n is an integer from about 10 - 60.

Further examples of preferred surfactants are the following:
The fluoroaliphatic polymeric esters known as FC 430 and FC 431, available from 3M. These are believed to be acrylic polymers having a fluorinated portion based on 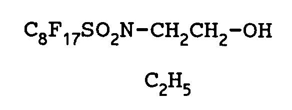
and a portion including an ethylene/propylene oxide block copolymer. These surfactants may be supplied as a 50:50 mixture with ethyl acetate, the latter compound being preferably removed before the surfactant is used in accordance with the present invention.

Other fluorinated surfactants produced by 3M that may be mentioned include FC 170c, FC 171 and FC 807. We particularly prefer surfactants which have both a fluorinated portion, especially a perfluorinated portion, and a hydrophilic portion, eg a portion based on an ethylene and/or propylene oxide.

Other fluorinated surfactants which may be mentioned are ethyl perfluorooctylsulphonamide, the linear perfluoropolyether known as Fomblin-M, perfluorodecalin and tris(1H,1H,5H-octafluoropentyl)phosphate. All of these are available from Fluorochem Ltd.

Mixtures of fluorinated surfactants may also be used, eg mixtures of two or more of the fluorinated surfactants listed above. Alternatively, mixtures may be used of one or more fluorinated surfactants with one or more of the surfactants conventionally used in aerosol compositions, eg CFC-pressurised compositions. Examples of such conventional surfactants are: natural oils, sorbitan oleates, eg monooleate and trioleate, sorbitan monolaurate, monoglycerides, eg glyceryl monooleate, monostearate and monolaurate, lecithins, oleic acid, etc.

Other surfactants and adjuvants that may be added include poloxamers and/or polyethylene glycols, eg PEG 1000 and PEG 1500.

In the present context, the term 'hydrofluorocarbon' is to be taken to mean a compound of general formula

CₓH_{y}F_{z}

in which x is an integer from 1 to 3, $\text{y+z=2x+2}$ and y and z are both at least 1.

Particular hydrofluorocarbons of interest are CF₃CFH₂ (Propellant 134a), CH₃CHF₂ (Propellant 152a) and CF₃CHFCF₃ (Propellant 227). We particularly prefer compositions including propellant 227.

In general the vapour pressure of the mixture should be in the range suitable and permitted for aerosol propellants. The vapour pressure may be varied by mixing one or more hydrofluorocarbons and/or some other suitable vapour pressure modifying agent in appropriate proportions.

We prefer the vapour pressure of the mixture to be in the range 20 to 100 psi, more preferably 40 to 80 psi, eg about 60 psi.

The amount of surfactant in the composition will generally be from about 0.01 to 10% by weight, more preferably from about 0.1 to 5%, eg about 1%.

The medicament may be in solid, particulate form (ie the composition may be a suspension), or the active ingredient may be dissolved in the propellant.

Medicaments which may be dispersed in the propellant mixture according to the invention include any medicaments which are conventionally administered by inhalation of a pressurised aerosol formulation. Such medicaments include drugs for use in the prophylactic or remedial treatment of reversible obstructive airways disease, eg drugs such as sodium cromoglycate, nedocromil sodium, inhaled steroids, eg beclomethasone dipropionate, fluticasone and tipredane, and bronchodilators, eg salbutamol, reproterol, terbutaline, formoterol, pirbuterol, isoprenaline, salmeterol, fenoterol and salts thereof, and anticholinergic agents such as ipratropium bromide and atropine.

Where the active ingredient is solid, it preferably has a particle size distribution such that a high proportion of the particles are of a size capable of penetrating deep into the lung. In particular, the active ingredient is preferably in a form having a mass median diameter of from 0.1 to 10 »m, more preferably from 0.1 to 4 »m, eg about 2 or 3 »m.

We prefer the medicament to have a mass median diameter in the range 0.01 to 10 microns, more preferably from 1 to 5 microns. The composition preferably comprises from 0.01 to 15, preferably from 0.1 to 10, and most preferably from 0.5 to 5% w/w medicament.

In producing the compositions according to the invention, a container equipped with a valve is filled with a propellant containing the finely-divided medicament. The container may first be charged with a weighed amount of medicament which has been ground to a predetermined particle size, or with a slurry of powder in the cooled liquid propellant. The container may alternatively be filled by introducing powder and propellant by the normal cold filling method, or a slurry of the powder in one component of the propellant may be placed in the container, the valve sealed in place, and the balance of the propellant then introduced by pressure filling through the valve nozzle. As a further alternative a bulk quantity of the total composition may be filled into the container through the valve.

The invention will now be illustrated, but in no way limited, by the following Example.

### Example

Compositions were prepared by cold filling of the ingredients into aluminium aerosol canisters which were then sealed by crimping a 50»l or 100»l aerosol valve in place.

The following combinations of micronised active ingredient, surfactant and propellant were used, removing solvent from the surfactant where necessary:
1.

| | |
|---|---|
| Nedocromil sodium | 0.200g |
| FC 431 | 0.061g |
| Propellant 134a | 11.979g |

2.

| | |
|---|---|
| Tipredane | 0.100g |
| FC 431 | 0.071g |
| Propellant 227 | 13.949g |

3.

| | |
|---|---|
| Sodium cromoglycate | 0.200g |
| FC 430 | 0.061g |
| Propellant 134a | 11.979g |

4.

| | |
|---|---|
| Sodium cromoglycate | 0.200g |
| FC 430 | 0.071g |
| Propellant 227 | 13.849g |

5.

| | |
|---|---|
| Nedocromil sodium | 0.200g |
| FC 430 | 0.061g |
| Propellant 134a | 11.979g |

6.

| | |
|---|---|
| Nedocromil sodium | 0.200g |
| FC 430 | 0.071g |
| Propellant 227 | 13.849g |

7.

| | |
|---|---|
| Salbutamol sulphate | 0.040g |
| FC 431 | 0.061g |
| Propellant 134a | 12.139g |

8.

| | |
|---|---|
| Fenoterol hydrobromide | 0.040g |
| FC 430 | 0.071g |
| Propellant 227 | 14.009g |

In all cases stable suspensions of the active ingredient in the propellant were obtained.

## Claims (Claims for the following Contracting State(s): AT, BE, CH, DE, DK, FR, GB, IT, LU, NL, SE)

1. A pressurised aerosol composition comprising a liquified hydrofluorocarbon propellant having dispersed therein a medicament; and a fluorinated surfactant dissolved in the propellant; characterised in that the solvent for the fluorinated surfactant consists essentially of the hydrofluorocarbon propellant.

2. A composition according to claim 1, wherein the surfactant is a non-ionic surfactant.

3. A composition according to claim 1 or 2, wherein the surfactant contains at least one (CF₂) group.

4. A composition according to any one of claims 1, 2 or 3, wherein the surfactant contains one or more ether or carboxylic ester linkages.

5. A composition according to any one of the preceding claims, wherein the surfactant contains at least one (CH₂) group.

6. A composition according to any one of the preceding claims, wherein the composition includes an additional surfactant selected from poloxamers and polyethylene glycols.

7. A composition according to any one of the preceding claims, wherein the propellant is CF₃CFH₂, CH₃CHF₂, CF₃CHFCF₃ or mixtures thereof.

8. A composition according to any one of the preceding claims, wherein the medicament is sodium cromoglycate, nedocromil sodium, beclomethasone diproprionate, fluticosone, tipredane, ipratropium bromide, atropine or a brochodilator selected from salbutamol, reproterol, terbutaline, fomoterol, pirbuterol, isoprenaline, salmeterol, fenoterol or a salt of any one thereof.

9. A composition according to any one of the preceding claims, comprising from 0.01 to 10% by weight of fluorinated surfactant.

10. A composition according to any one of the preceding claims, comprising from 0.01 to 15% w/w medicament.

## Claims (Claims for the following Contracting State(s): ES, GR)

1. A process for the production of a pressurised aerosol composition comprising a liquified hydrofluorocarbon propellant having dispersed therein a medicament; and a fluorinated surfactant dissolved in the propellant; characterised in that the solvent for the fluorinated surfactant consists essentially of the hydrofluorocarbon propellant; which process comprises mixing the medicament and the surfactant with the propellant.

2. A process according to claim 1, wherein the surfactant is a non-ionic surfactant.

3. A process according to claim 1 or 2, wherein the surfactant contains at least one (CF₂) group.

4. A process according to any one of claims 1, 2 or 3, wherein the surfactant contains one or more ether or carboxylic ester linkages.

5. A process according to any one of the preceding claims, wherein the surfactant contains at least one (CH₂) group.

6. A process according to any one of the preceding claims, wherein the composition includes an additional surfactant selected from poloxamers and polyethylene glycols.

7. A process according to any one of the preceding claims, wherein the propellant is CF₃CFH₂, CH₃CHF₂, CF₃CHFCF₃ or mixtures thereof.

8. A process according to any one of the preceding claims, wherein the medicament is sodium cromoglycate, nedocromil sodium, beclomethasone dipropionate, fluticasone, tipredane, ipratropium bromide, atropine or a bronchodilator selected from salbutamol, reproterol, terbutaline, formoterol, pirbuterol, isoprenaline, salmeterol, fenoterol or a salt of any one thereof.

9. A process according to any one of the preceding claims, wherein the composition comprises from 0.01 to 10% by weight of fluorinated surfactant.

10. A process according to any one of the preceding claims, wherein the composition comprises from 0.01 to 15% w/w medicament.

## Patentansprüche (Patentansprüche für folgende(n) Vertragsstaat(en): AT, BE, CH, DE, DK, FR, GB, IT, LU, NL, SE)

1. Unter Druck befindliche Aerosol-Zusammensetzung, umfassend ein verflüssigtes Fluorkohlenwasserstoff-Treibmittel mit einem darin dispergierten Medikament; und ein im Treibmittel gelöstes fluoriertes grenzflächenaktives Agens; dadurch gekennzeichnet, daß das Lösungsmittel für das fluorierte grenzflächenaktive Agens im wesentlichen aus dem Fluorkohlenwasserstoff-Treibmittel besteht.

2. Zusammensetzung nach Anspruch 1, wobei das grenzflächenaktive Agens ein nicht-ionisches grenzflächenaktives Agens ist.

3. Zusammensetzung nach Anspruch 1 oder 2, wobei das grenzflächenaktive Agens mindestens eine (CF₂)-Gruppe enthält.

4. Zusammensetzung nach einem der Ansprüche 1, 2 oder 3, wobei das grenzflächenaktive Agens eine oder mehrere Ether- oder Carbonsäureester-Bindungen enthält.

5. Zusammensetzung nach einem der vorhergehenden Ansprüche, wobei das grenzflächenaktive Agens mindestens eine (CH₂)-Gruppe enthält.

6. Zusammensetzung nach einem der vorhergehenden Ansprüche, wobei die Zusammensetzung ein zusätzliches grenzflächenaktives Agens ausgewählt aus Poloxameren und Polyethylenglykolen enthält.

7. Zusammensetzung nach einem der vorhergehenden Ansprüche, wobei das Treibmittel CF₃CFH₂, CH₃CHF₂, CF₃CHFCF₃ oder Mischungen davon ist (sind).

8. Zusammensetzung nach einem der vorhergehenden Ansprüche, wobei das Medikament Natriumchromglykat, Nedochromil-Natrium, Beclomethason-dipropionat, Fluticoson, Tipredan, Ipratropiumbromid, Atropin oder ein Bronchodilatationsmittel ausgewählt aus Salbutamol, Reproterol, Terbutalin, Fomoterol, Pirbuterol, Isoprenalin, Salmeterol, Fenoterol oder ein Salz von irgendeinem derselben ist.

9. Zusammensetzung nach einem der vorhergehenden Ansprüche, welche von 0,01 bis 10 Gew.-% des fluorierten grenzflächenaktiven Agens umfaßt.

10. Zusammensetzung nach einem der vorhergehenden Ansprüche, welche von 0,01 bis 15 % Gew./Gew. Medikament umfaßt.

## Patentansprüche (Patentansprüche für folgende(n) Vertragsstaat(en): ES, GR)

1. Verfahren zur Herstellung einer unter Druck befindlichen Aerosol-Zusammensetzung, welche ein verflüssigtes Fluorkohlenwasserstoff-Treibmittel mit einem darin dispergierten Medikament; und ein im Treibmittel gelöstes fluoriertes grenzflächenaktives Agens umfaßt; dadurch gekennzeichnet, daß das Lösungsmittel für das fluorierte grenzflächenaktive Agens im wesentlichen aus dem Fluorkohlenwasserstoff-Treibmittel besteht; welches Verfahren das Mischen des Medikaments und des grenzflächenaktiven Agens mit dem Treibmittel umfaßt.

2. Verfahren nach Anspruch 1, wobei das grenzflächenaktive Agens ein nicht-ionisches grenzflächenaktives Agens ist.

3. Verfahren nach Anspruch 1 oder 2, wobei das grenzflächenaktive Agens mindestens eine (CF₂)-Gruppe enthält.

4. Verfahren nach einem der Ansprüche 1, 2 oder 3, wobei das grenzflächenaktive Agens einen oder mehrere Ether- oder Carbonsäureester-Bindungen enthält.

5. Verfahren nach einem der vorhergehenden Ansprüche, wobei das grenzflächenaktive Agens mindestens eine (CH₂)-Gruppe enthält.

6. Verfahren nach einem der vorhergehenden Ansprüche, wobei die Zusammensetzung ein zusätzliches grenzflächenaktives Agens ausgewählt aus Poloxameren und Polyethylenglykolen enthält.

7. Verfahren nach einem der vorhergehenden Ansprüche, wobei das Treibmittel CF₃CFH₂, CH₃CHF₂, CF₃CHFCF₃ oder Mischungen davon ist (sind).

8. Verfahren nach einem der vorhergehenden Ansprüche, wobei das Medikament Natriumchromglykat, Nedocromil-Natrium, Beclomethason-dipropionat, Fluticason, Tipredan, Ipratropiumbromid, Atropin oder ein Bronchodilatationsmittel ausgewählt aus Salbutamol, Reproterol, Terbutalin, Formoterol, Pirbuterol, Isoprenalin, Salmeterol, Fenoterol oder ein Salz von irgendeinem derselben ist.

9. Verfahren nach einem der vorhergehenden Ansprüche, wobei die Zusammensetzung von 0,01 bis 10 Gew.-% des fluorierten grenzflächenaktiven Agens umfaßt.

10. Verfahren nach einem der vorhergehenden Ansprüche, wobei die Zusammensetzung von 0,01 bis 15 % Gew./Gew. Medikament umfaßt.

## Revendications (Revendications pour l'(les) Etat(s) contractant(s) suivant(s): AT, BE, CH, DE, DK, FR, GB, IT, LU, NL, SE)

1. Composition d'un aérosol pressurisé comprenant un propulseur hydrofluorocarboné, liquéfié, qui contient un médicament y dispersé, et un tensioactif fluoré dissous dans le propulseur, caractérisée en ce que le solvant du tensioactif fluoré consiste essentiellement en le propulseur hydrofluorocarboné.

2. Composition suivant la revendication 1, dans laquelle le tensioactif est un tensioactif non ionique.

3. Composition suivant la revendication 1 ou 2, dans laquelle le tensioactif contient au moins un radical (CF₂).

4. Composition suivant l'une quelconque des revendications 1, 2 et 3, dans laquelle le tensioactif contient une ou plusieurs liaisons éther ou ester carboxylique.

5. Composition suivant l'une quelconque des revendications précédentes, dans laquelle le tensioactif contient au moins un radical (CH₂).

6. Composition suivant l'une quelconque des revendications précédentes, où la composition contient un tensioactif supplémentaire choisi parmi les polyoxamères et les polyéthylèneglycols.

7. Composition suivant l'une quelconque des revendications précédentes, dans laquelle le propulseur est CF₃CFH₂, CH₃CHF₂, CF₃CHFCF₃ ou des mélanges de ceux-ci.

8. Composition suivant l'une quelconque des revendications précédentes, dans laquelle le médicament est le cromoglycate de sodium, le nédocromil sodique, le dipropionate de béclométhasone, la fluticasone, le tiprédane, le bromure d'ipratropium, l'atropine ou un bronchodilatateur choisi parmi le salbutamol, le réprotérol, la terbutaline, le formotérol, le pirbutérol, l'isoprénaline, le salmétérol, le fénotérol ou un sel de l'un quelconque de ceux-ci.

9. Composition suivant l'une quelconque des revendications précédentes, comprenant de 0,01 à 10% en poids du tensioactif fluoré.

10. Composition suivant l'une quelconque des revendications précédentes, comprenant de 0,01 à 15% p/p du médicament.

## Revendications (Revendications pour l'(les) Etat(s) contractant(s) suivant(s): ES, GR)

1. Procédé de préparation d'une composition d'un aérosol pressurisé comprenant un propulseur hydrofluorocarboné, liquéfié, qui contient un médicament y dispersé, et un tensioactif fluoré dissous dans le propulseur, caractérisé en ce que le solvant du tensioactif fluoré consiste essentiellement en le propulseur hydrofluorocarboné, le procédé comprend le mélange du médicament et du tensioactif au propulseur.

2. Procédé suivant la revendication 1, dans lequel le tensioactif est un tensioactif non ionique.

3. Procédé suivant la revendication 1 ou 2, dans lequel le tensioactif contient au moins un radical (CF₂).

4. Procédé suivant l'une quelconque des revendications 1, 2 et 3, dans lequel le tensioactif contient une ou plusieurs liaisons éther ou ester carboxylique.

5. Procédé suivant l'une quelconque des revendications précédentes, dans lequel le tensioactif contient au moins un radical (CH₂).

6. Procédé suivant l'une quelconque des revendications précédentes, dans lequel la composition contient un tensioactif supplémentaire choisi parmi les polyoxamères et les polyéthylèneglycols.

7. Procédé suivant l'une quelconque des revendications précédentes, dans lequel le propulseur est CF₃CFH₂, CH₃CHF₂, CF₃CHFCF₃ ou des mélanges de ceux-ci.

8. Procédé suivant l'une quelconque des revendications précédentes, dans lequel le médicament est le cromoglycate de sodium, le nédocromil sodique, le dipropionate de béclométhasone, la fluticasone, le tiprédane, le bromure d'ipratropium, l'atropine ou un bronchodilatateur choisi parmi le salbutamol, le réprotérol, la terbutaline, le formotérol, le pirbutérol, l'isoprénaline, le salmétérol, le fénotérol ou un sel de l'un quelconque de ceux-ci.

9. Procédé suivant l'une quelconque des revendications précédentes, dans lequel la composition comprend de 0,01 à 10% en poids du tensioactif fluoré.

10. Procédé suivant l'une quelconque des revendications précédentes, dans lequel la composition comprend de 0,01 à 15% p/p du médicament.
